# EUROPEAN PATENT SPECIFICATION

(11) **EP 3 790 444 B1**
(45) Date of publication and mention of the grant of the patent: **28.01.2026**
(21) Application number: 19727197.6
(22) Date of filing: 09.05.2019
(51) Int. Cl.: A61B 1/00, A61B 1/06, A61B 1/07, A61B 1/12, G02B 6/42, G02B 23/24

(54) **FLEXIBLE LIGHT GUIDE AND HEAT SINK FOR ENDOSCOPIC SYSTEMS**
FLEXIBLE LICHTLEITER UND KÜHLKÖRPER FÜR ENDOSKOPISCHE SYSTEME
GUIDE DE LUMIÈRE FLEXIBLE ET DISSIPATEUR THERMIQUE POUR SYSTÈMES ENDOSCOPIQUES

(30) Priority: 09.05.2018 US 201862668979 P; 11.05.2018 US 201862670242 P; 21.09.2018 US 201862734691 P
(43) Date of publication of application: 17.03.2021
(73) Proprietor: CONMED Corporation, Largo, FL 33773 (US)
(72) Inventor: MCLEAN, Edward, Billerica, MA 01821 (US); SZABO, Michael, St. Petersburg, FL 33703 (US)
(74) Representative: Strehl & Partner mbB
(86) International application number: PCT/US2019/031480
(87) International publication number: WO 2019/217652

(56) References cited:
- WO-A1-97/21130
- JP-A- 2000 310 739
- US-A- 5 735 794
- US-A1- 2006 183 977
- US-A1- 2011 092 772
- US-B1- 8 622 896
- US-B2- 7 668 450

## Description

### BACKGROUND OF THE INVENTION

### 1. FIELD OF THE INVENTION

The present invention to endoscopic light guides and, more specifically, to a endoscopic light guide having a flexible heat sink for the light emitting diode.

### 2. DESCRIPTION OF THE RELATED ART

In the field of endoscopy surgery it is necessary to direct visible light into the human body to illuminate various surgical sites. Traditionally this is achieved by attaching the distal end a fiber optic light guide to the fiber optic light post of an endoscope. The opposite proximal end of the light guide is connected to an electronic light source. Typically the light source is an AC voltage powered electronic enclosure that contains a high output lamp with a fiber optic cable that couples to the light post of the endoscope for optimized light transmission. One of the characteristics of existing fiber optic light guides is that they are flexible and can be manipulated easily by the surgeon when adjusting or rotating the endoscope to view different surgical sites within the body.

US 5735794 A discloses a light illuminating supply unit having a lamp at the distal end of flexible tube, wherein the supply unit is configured to be coupled to an endoscope light port and the flexible tube contains electric wires to power the lamp. The supply unit includes a transparent plate for light transmission from the lamp to the endoscope light port.

A limitation of existing fiber optic light guides is that their performance deteriorates over use due to the properties of the materials used in their construction. The light guide is comprised of many individual glass fiber optic strands that make up an arrayed bundle. An outer jacket surrounds this glass strand bundle to provide protection from the environment that the device will be subjected to during use, cleaning, and reprocessing (sterilization). The individual fiber strands are made from drawn glass that is prone to breakage during use. When an individual fiber breaks anywhere along its length, it no longer is able to transmit light and hence the overall light transmission from the light source output will be diminished by a percentage. Conventional attempts to overcome these problems involve the use of a battery powered LED source that attaches to the light post of the endoscope. These devices, however, as limited to available battery life and thus have short usage times between charging operation and or battery replacement. Accordingly, there is a need in the art for an approach that can provide illumination to the light port of an endoscope for longer periods of time while retaining the desired flexibility of the cable.

### BRIEF SUMMARY OF THE INVENTION

The above problem is solved by a flexible electronic light guide as set forth in the appended claims.

The present invention comprises a flexible electronic light guide for an endoscope that includes a heat sink for the light emitting diode module of the light guide. The heat sink is designed so that the electronic light guide retains the flexible form factor desired by surgeons while shunting heat from the light source so that the components to be touched by a surgeon do not overheat. The electronic light guide does not use glass fiber optics and relies on a calibrated fixed illumination output that does not decrease over the life of the product. The electronic light guide also reduces the number of coupled optical surfaces that are present when using traditional fiber optic light guides. The connection made at the light source at the proximal end of the light guide is eliminated when using the electronic light guide because the LED is positioned directly at the scope post. By eliminating this optical connection, the associated surface loss is also eliminated which in turn increases light output.

More specifically, the present invention is flexible electronic light guide has a light emitting diode module having a light emitting diode (LED) within a housing. A power cable is coupled to the LED to provide power to the LED. A flexible tube adjacent to the light emitting diode module extends from the LED and about the power cable to shunt heat away from the light emitting diode module.

In an embodiment, the flexible tube may comprise a solid metal tube that is perforated according to a predetermined pattern that allows the solid tube to be flexible. In another embodiment, the flexible tube may comprise a braided wire jacket. The braided wire jacket may enclose a series of metal balls that are in contact with each other. The flexible tube may also comprise a plurality of metals. In a further embodiment, the flexible tube may comprise a hollow tube be coupled to a fan at one end and a manifold at the other end of the tube that is positioned about the light emitting diode module. The manifold may have a front face defining series of openings. The fan may be positioned within a base unit to which the flexible tube is attached. In an additional embodiment, the flexible tube may comprise a hollow tube coupled to a fluid reservoir. The hollow tube may include at least one valve configured to only allow the flow of fluid in one predetermined direction. In yet another embodiment, the flexible tube may comprise a linkage formed from a series of components held in contact with each other by a spring. In yet an additional embodiment, the flexible tube may comprise a hollow tube packed with a plurality of a plurality of metal balls. The plurality of a plurality of metal balls may have a diameter between .0625 millimeters and 2.00 millimeters.

### BRIEF DESCRIPTION OF THE SEVERAL VIEWS OF THE DRAWING(S)

The present invention will be more fully understood and appreciated by reading the following Detailed Description in conjunction with the accompanying drawings, in which:
FIG. 1 is a perspective view of an endoscope having a flexible light guide according to the present invention;
FIG. 2 is a cross-sectional view of a flexible light guide according to the present invention;
FIG. 3 is an exploded view of the flexible light guide of FIG. 2 according to the present invention;
FIG. 4 is an exemplary pattern that may be etched into a solid metal tube to allow the tube to be flexible according to the present invention;
FIG. 5 is schematic of a flexible heat sink formed from multiple metals coupled together sink according to the present invention;
FIG. 6 is a flexible heat sink formed from a braided wire mesh heat sink for a flexible light guide according to the present invention.
FIG. 7 is flexible heat sink formed from a braided wire jacket enclosing a series of metal balls according to the present invention;
FIG. 8 is flexible heat sink formed from a braided wire jacket enclosing a series of metal balls according to the present invention; and
FIG. 9 is flexible heat sink formed from a hollow tube enclosing a plurality of metal balls according to the present invention.

### DETAILED DESCRIPTION OF THE INVENTION

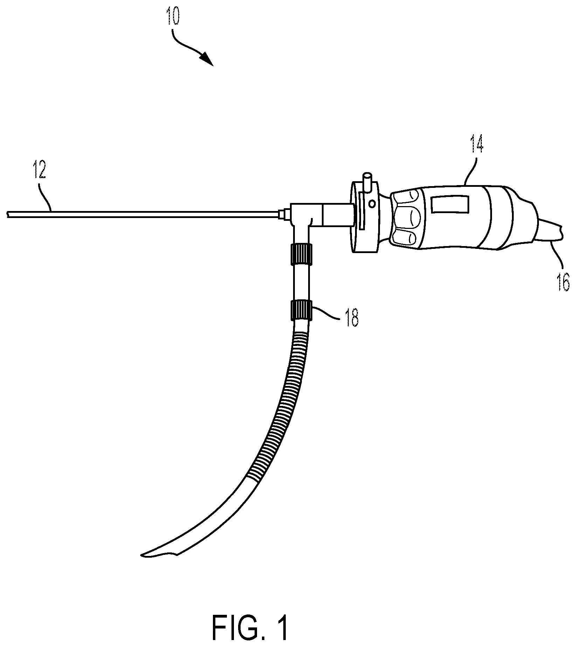

Referring to the figures, wherein like numeral refer to like parts throughout, there is seen in FIG. 1 an endoscope 10 having an insertion tube 12 that allows imaging components within a handpiece 14 to capture optical images of the field of view of the opposing end of insertion tube 12. A flexible shaft 16 extends from handpiece 14 to transmit images captured by the imaging components within handpiece 14 to a remotely positioned display, such as a view screen or monitor. An electronic light guide 18 is coupled transversely to insertion tube 12 at a light input port 20 of endoscope 10.

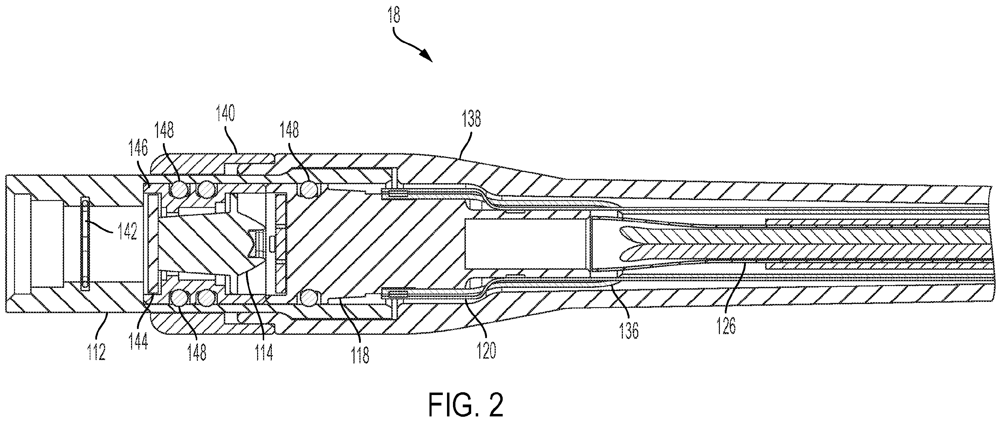

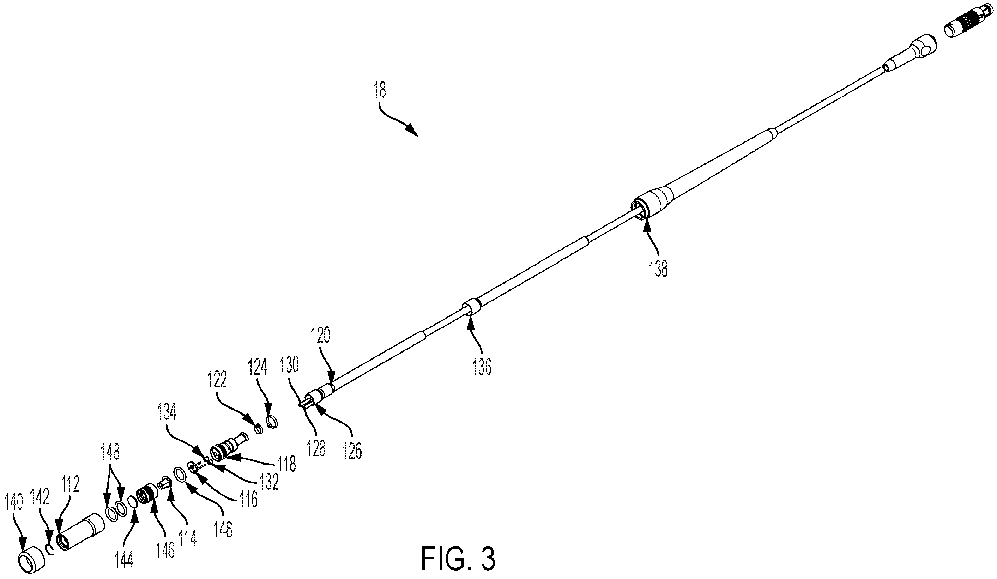

Referring to FIGS. 2 and 3, electronic light guide 18 comprises a body 112 housing a lens 114 and an LED 116 mounted to a printed circuit board and positioned behind lens 114. A window 144 is positioned in front of lens 114 to protect lens 114 and allow for light from LED 116 to be emitted from electronic light guide 18 to the light port of endoscope 10. It should be recognized that LED 116, lens 114 and window 140 may packaged into a cell 146 to form a discrete module that can be integrated into electronic light guide 18 in the manner described herein. A solid heat sink 118 is positioned adjacently to LED 116 for removal of heat generated when LED 116 is activated and extends rearwardly out of body 112. Solid heat sink 118 may be a copper body and can include fins for improved dispersal of accumulated heat. Solid heat sink 118 may be adhered to LED 116 using a metallic epoxy, such as silver epoxy, to encourage the transfer of heat away from LED 116. A flexible heat sink 120, such as a braided copper mesh, is attached directly to rigid heat sink 118 by one of more band clamps 122 and 124. A cable 126 extends within flexible heat sink 120 and includes a pair of wires 128 and 130 for powering LED 116. Wires 128 and 130 extend through solid heat sink 118 and are insulated therefrom by a pair of corresponding insulator sleeves 132 and 134, respectively. A heat shrink 136 may be positioned over an intermediate portion of flexible heat sink 120 to keep the braided mesh compressed. A boot 138 for providing strain relief is positioned over the components as well as at least a portion of body 112 and is held in place by a ring 140 attached over the forward end of body 112 into engagement with boot 138. A fitting 142 may be positioned inside the forward end of body 112 for selectively coupling and decoupling electronic light guide 18 to light port 20 of endoscope 10. O-rings 148 may be included in various locations to seal the internal components of electronic light guide 18 from ingress during use and allow for sterilization of electronic light guide 18 after use.

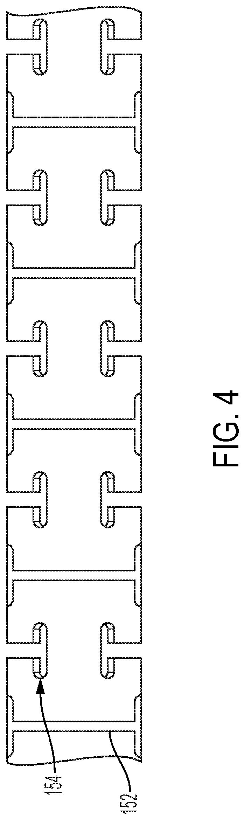

Although flexible heat sink 120 is preferably a braided wire mesh, the present invention includes other approaches that provide the requisite flexibility and heat transfer down a portion of the length of electronic light guide 18. Referring to FIG. 4, for example, a flexible heat sink 150 may comprise a flexible shaft 152 that is coupled to rigid heat sink 118. Flexible shaft 152 may be formed by laser cutting a solid tube formed from a thermally conductive material, such as metal, to remove material from shaft 152 according to a predetermined pattern 154 that will allow shaft 152 to flex in three dimensions. One end of shaft 152 is coupled to rigid heat sink 118 to draw heat away from LED 116 while allowing for movement of endoscope 10 via the flexing of shaft 152.

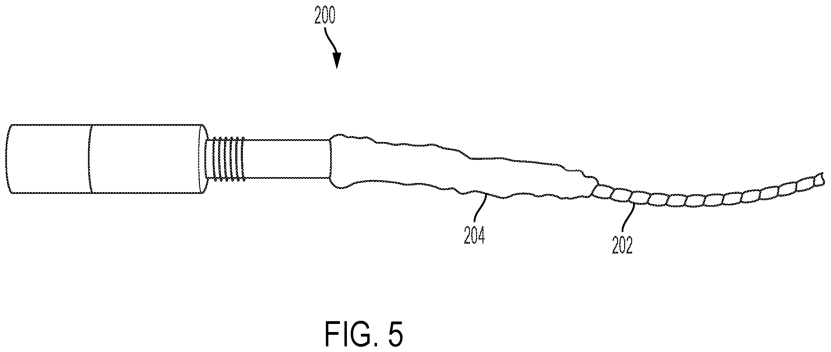

Referring to FIG. 5, a flexible heat sink 200 according to the present invention may comprise a central electrical cable 202 that is surrounded by multiple thermally conductive materials 204 placed into contact with rigid heat sink 118..

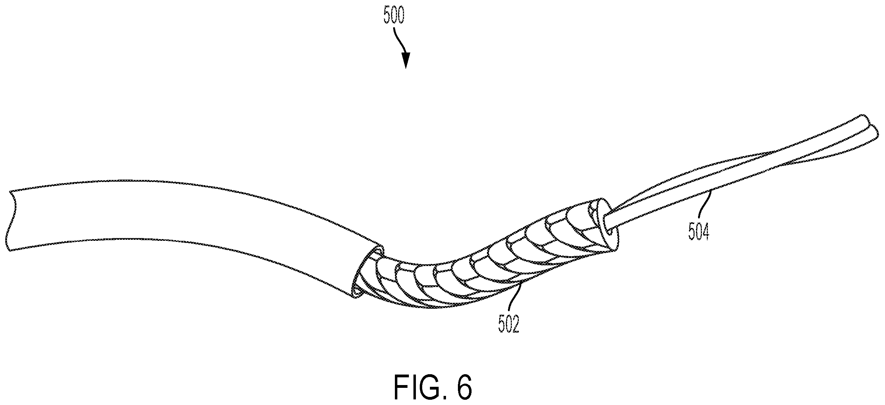

Referring to FIG. 6, a flexible heat sink 500 according to the present invention may comprise a braided jacket 502 surrounding the electrical wiring 504 for rigid heat sink 118. Braided jacket 502 is formed from one or more materials that are thermally conductive. Braided jacket 502 is placed into contact with rigid heat sink 118 to remove heat therefrom.

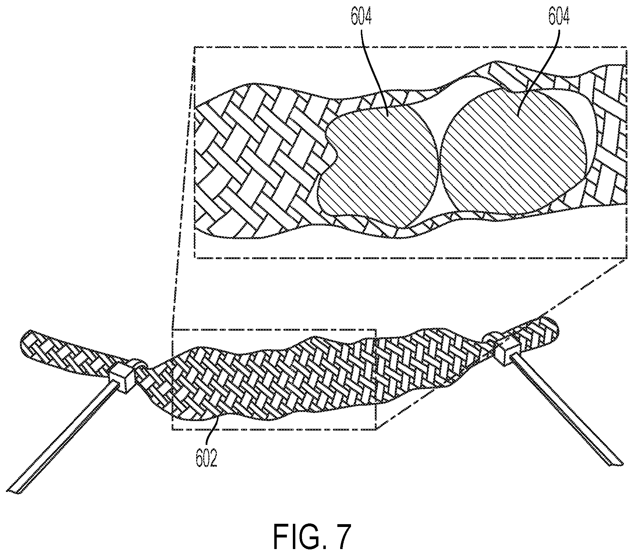

Referring to FIG. 7, a flexible heat sink 600 according to the present invention may comprise a braided jacket 602 surrounding the electrical wiring for rigid heat sink 118. A series of balls 604 are packed tightly within braided jacket 602. Braided jacket 602 and balls 604 are formed from one or more materials that are thermally conductive, such as metal. Braided jacket 602 is placed into contact with rigid heat sink 118 to remove heat therefrom with balls 604 providing additional heat collectors.

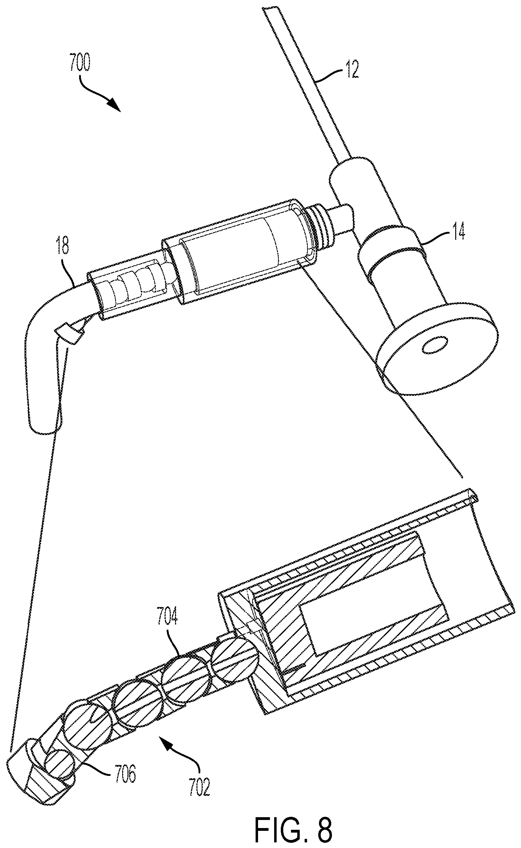

Referring to FIG. 8, a flexible heat sink 700 according to the present invention may comprise a linkage 702 formed from a series of components 704 compressed tightly together by a biasing element 706, such as a spring. Tight compression of components 704 allows for ready transmission of heat and for flexible movement when an endoscope including heat sink 700 and rigid heat sink 118 is used.

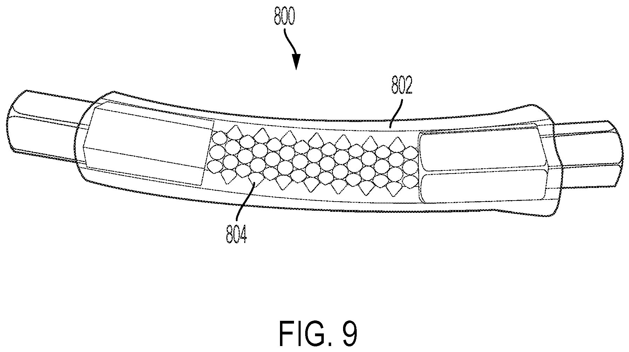

Referring to FIG. 9, a flexible heat sink 800 according to the present invention may comprise a flexible jacket 802 surrounding the electrical wiring for rigid heat sink 118. A series of balls 804 are packed tightly within flexible jacket 802. Flexible jacket 802 and balls 804 are formed from one or more materials that are thermally conductive. Flexible jacket 602 is placed so that balls 604 are in contact with rigid heat sink 118 to remove heat therefrom. Balls 804 may be as small as grains of sand (between .0625 millimeters and 2.00 millimeters) or larger, such as ball bearings.

## Claims

1. A flexible electronic light guide configured to be coupled to a light input port (20) of an endoscope, comprising
a housing (112);
a window (144); and
a power cable (126),
the housing (112) comprises a light emitting diode (116) and a lens (114) with the window positioned in front of the lens (114) for light from the light emitting diode (116) to be emitted to the light input port (20) of the endoscope,
the power cable (126) is coupled to the light emitting diode (116), and
a flexible tube (120, 150, 200, 500, 600, 700, 800) is provided adjacent to the light emitting diode (116) and extending about the power cable (126) to shunt heat away from the light emitting diode (116).

2. The flexible electronic light guide of claim 1, wherein the flexible tube (150) comprises a solid metal tube (152) that is perforated according to a predetermined pattern (154) that allows the solid metal tube to be flexible.

3. The flexible electronic light guide of claim 1, wherein the flexible tube (500, 600) comprises a braided wire jacket (502, 602).

4. The flexible electronic light guide of claim 3, wherein the braided wire jacket (602) encloses a plurality of metal balls (604) that are in contact with each other.

5. The flexible electronic light guide of claim 3, wherein the flexible tube (200) comprises a plurality of metals.

6. The flexible electronic light guide of claim 1, wherein the flexible tube comprises a hollow tube and the flexible electronic light guide further comprises a fan coupled to an end of the tube and a manifold coupled to another end of the tube and positioned about the light emitting diode module.

7. The flexible electronic light guide of claim 6, wherein the manifold comprises a front face defining series of openings.

8. The flexible electronic light guide of claim 7, wherein the fan is positioned within a base unit to which the flexible tube is attached.

9. The flexible electronic light guide of claim 1, wherein the flexible tube comprises a hollow tube coupled to a fluid reservoir.

10. The flexible electronic light guide of claim 9, wherein the hollow tube includes at least one valve configured to only allow a flow of fluid in one predetermined direction.

11. The flexible electronic light guide of claim 1, wherein the flexible tube (700) comprises a linkage (702) formed from a series of components (704) held in contact with each other by a spring (706).

12. The flexible electronic light guide of claim 1, wherein the flexible tube (800) comprises a hollow tube packed with a plurality of a plurality of metal balls (804).

13. The flexible electronic light guide of claim 12, wherein the plurality of a plurality of metal balls (804) have a diameter between .0625 millimeters and 2.00 millimeters.

## Patentansprüche

1. Flexibler elektronischer Lichtleiter, der dazu eingerichtet ist, an einen Lichteingangsanschluss (20) eines Endoskops gekoppelt zu werden, umfassend
ein Gehäuse (112);
ein Fenster (144); und
ein Stromkabel (126),
wobei das Gehäuse (112) eine Leuchtdiode (116) und eine Linse (114) umfasst, wobei das Fenster vor der Linse (114) positioniert ist, damit Licht von der Leuchtdiode (116) zum Lichteingangsanschluss (20) des Endoskops emittiert wird,
das Stromkabel (126) mit der Leuchtdiode (116) gekoppelt ist, und
eine flexible Röhre (120, 150, 200, 500, 600, 700, 800) angrenzend an die Leuchtdiode (116) vorgesehen ist und sich um das Stromkabel (126) erstreckt, um Wärme von der Leuchtdiode (116) abzuhalten.

2. Flexibler elektronischer Lichtleiter nach Anspruch 1, wobei die flexible Röhre (150) eine feste Metallröhre (152) umfasst, die gemäß einem vorbestimmten Muster (154) perforiert ist, das es der festen Metallröhre ermöglicht, flexibel zu sein.

3. Flexibler elektronischer Lichtleiter nach Anspruch 1, wobei die flexible Röhre (500, 600) einen geflochtenen Drahtmantel (502, 602) umfasst.

4. Flexibler elektronischer Lichtleiter nach Anspruch 3, wobei der geflochtene Drahtmantel (602) mehrere Metallkugeln (604) umschließt, die miteinander in Kontakt stehen.

5. Flexibler elektronischer Lichtleiter nach Anspruch 3, wobei die flexible Röhre (200) mehrere Metalle umfasst.

6. Flexibler elektronischer Lichtleiter nach Anspruch 1, wobei die flexible Röhre eine hohle Röhre umfasst und der flexible elektronische Lichtleiter ferner einen Lüfter, der an ein Ende der Röhre gekoppelt ist, und einen Verteiler, der an ein anderes Ende der Röhre gekoppelt und um das Leuchtdiodenmodul herum positioniert ist, umfasst.

7. Flexibler elektronischer Lichtleiter nach Anspruch 6, wobei der Verteiler eine Vorderseite umfasst, die eine Reihe von Öffnungen definiert.

8. Flexibler elektronischer Lichtleiter nach Anspruch 7, wobei der Lüfter innerhalb einer Basiseinheit positioniert ist, an der die flexible Röhre befestigt ist.

9. Flexibler elektronischer Lichtleiter nach Anspruch 1, wobei die flexible Röhre eine hohle Röhre umfasst, die mit einem Fluidreservoir gekoppelt ist.

10. Flexibler elektronischer Lichtleiter nach Anspruch 9, wobei die hohle Röhre mindestens ein Ventil umfasst, das dazu eingerichtet ist, einen Fluidstrom in einer vorbestimmten Richtung zuzulassen.

11. Flexibler elektronischer Lichtleiter nach Anspruch 1, wobei die flexible Röhre (700) eine Verbindung (702) umfasst, die aus einer Reihe von Komponenten (704) gebildet ist, die durch eine Feder (706) miteinander in Kontakt gehalten werden.

12. Flexibler elektronischer Lichtleiter nach Anspruch 1, wobei die flexible Röhre (800) eine hohle Röhre umfasst, die mit mehreren Metallkugeln (804) gefüllt ist.

13. Flexibler elektronischer Lichtleiter nach Anspruch 12, wobei die mehreren Metallkugeln (804) einen Durchmesser zwischen 0,0625 Millimetern und 2,00 Millimetern aufweisen.

## Revendications

1. Guide de lumière électronique flexible configuré pour être couplé à un orifice (20) d'entrée de lumière d'un endoscope, comprenant
un boîtier (112) ;
une fenêtre (144) ; et
un câble (126) d'alimentation électrique,
le boîtier (112) comprend une diode électroluminescente (116) et une lentille (114) avec la fenêtre positionnée devant la lentille (114) pour que la lumière provenant de la diode électroluminescente (116) soit émise jusqu'à l'orifice (20) d'entrée de lumière de l'endoscope,
le câble (126) d'alimentation électrique est couplé à la diode électroluminescente (116), et
un tube flexible (120, 150, 200, 500, 600, 700, 800) est prévu adjacent à la diode électroluminescente (116) et s'étendant autour du câble (126) d'alimentation électrique pour éloigner une chaleur de la diode électroluminescente (116).

2. Guide de lumière électronique flexible selon la revendication 1, dans lequel le tube flexible (150) comprend un tube de métal plein (152) qui est perforé selon un motif prédéterminé (154) qui permet que le tube de métal plein soit flexible.

3. Guide de lumière électronique flexible selon la revendication 1, dans lequel le tube flexible (500, 600) comprend une gaine de fil tressée (502, 602).

4. Guide de lumière électronique flexible selon la revendication 3, dans lequel la gaine de fil tressée (602) renferme une pluralité de billes métalliques (604) qui sont en contact les unes avec les autres.

5. Guide de lumière électronique flexible selon la revendication 3, dans lequel le tube flexible (200) comprend une pluralité de métaux.

6. Guide de lumière électronique flexible selon la revendication 1, dans lequel le tube flexible comprend un tube creux et le guide de lumière électronique flexible comprend en outre un ventilateur couplé à une extrémité du tube et un collecteur couplé à une autre extrémité du tube et positionné autour du module de diode électroluminescente.

7. Guide de lumière électronique flexible selon la revendication 6, dans lequel le collecteur comprend une face avant définissant une série d'ouvertures.

8. Guide de lumière électronique flexible selon la revendication 7, dans lequel le ventilateur est positionné à l'intérieur d'une unité de base à laquelle le tube flexible est fixé.

9. Guide de lumière électronique flexible selon la revendication 1, dans lequel le tube flexible comprend un tube creux couplé à un réservoir de fluide.

10. Guide de lumière électronique flexible selon la revendication 9, dans lequel le tube creux inclut au moins une valve configurée pour ne permettre un écoulement de fluide que dans un sens prédéterminé.

11. Guide de lumière électronique flexible selon la revendication 1, dans lequel le tube flexible (700) comprend une liaison (702) constituée d'une série de composants (704) maintenus en contact les uns avec les autres par un ressort (706).

12. Guide de lumière électronique flexible selon la revendication 1, dans lequel le tube flexible (800) comprend un tube creux garni avec une pluralité d'une pluralité de billes métalliques (804).

13. Guide de lumière électronique flexible selon la revendication 12, dans lequel la pluralité d'une pluralité de billes métalliques (804) ont un diamètre entre 0,0625 millimètre et 2,00 millimètres.
